(19) Europäisches Patentamt / European Patent Office / Office européen des brevets

(11) **EP 2 019 865 B1**

(12) **EUROPEAN PATENT SPECIFICATION**

(45) Date of publication and mention
of the grant of the patent:
**12.03.2014 Bulletin 2014/11**

(21) Application number: **07732919.1**

(22) Date of filing: **22.05.2007**

(51) Int Cl.:
*C12N 9/96* *(2006.01)*    *A61K 39/39* *(2006.01)*

(86) International application number:
**PCT/GB2007/001898**

(87) International publication number:
**WO 2007/135425 (29.11.2007 Gazette 2007/48)**

(54) **STABLE VACCINE FORMULATION**

STABILE IMPFSTOFFFORMULIERUNG

FORMULE DE VACCIN STABLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.05.2006 GB 0610140**
**03.07.2006 PCT/GB2006/002470**

(43) Date of publication of application:
**04.02.2009 Bulletin 2009/06**

(73) Proprietor: **Arecor Limited**
**Cambridge CB4 OFE (GB)**

(72) Inventor: **JEZEK, Jan**
**Sharnbrook**
**Bedfordshire MK44 1LQ (GB)**

(74) Representative: **Teuten, Andrew John et al**
**Sagittarius IP**
**Three Globeside**
**Fieldhouse Lane**
**Marlow, Buckinghamshire SL7 1HZ (GB)**

(56) References cited:
**EP-A- 1 314 437      WO-A-03/009869**
**WO-A-03/021258      WO-A-2007/003936**

- **ELCOCK A H: "Realistic modeling of the denatured states of proteins allows accurate calculations of the ph dependence of protein stability" JOURNAL OF MOLECULAR BIOLOGY, LONDON, GB, vol. 294, no. 4, 10 December 1999 (1999-12-10), pages 1051-1062, XP004461830 ISSN: 0022-2836**
- **ALEXOV EMIL: "Numerical calculations of the pH of maximal protein stability. The effect of the sequence composition and three-dimensional structure." EUROPEAN JOURNAL OF BIOCHEMISTRY, vol. 271, no. 1, January 2004 (2004-01), pages 173-185, XP002398496 ISSN: 0014-2956**
- **ANTOSIEWICZ JAN ET AL: "Prediction of pH-dependent properties of proteins" JOURNAL OF MOLECULAR BIOLOGY, vol. 238, no. 3, 1994, pages 415-436, XP002398497 ISSN: 0022-2836**
- **JAN M ET AL: "ADSORPTION OF GLOBULAR PROTEINS TO VACCINE ADJUVANTS", JOURNAL OF BIOCHEMISTRY AND MOLECULAR BIOLOGY, KOREAN SOCIETY FOR BIOCHEMISTRY AND MOLECULAR BIOLOGY, KR, vol. 30, no. 5, 30 September 1997 (1997-09-30), pages 346-351, XP009005775, ISSN: 1225-8687**
- **IYER S ET AL: "Mechanism of adsorption of hepatitis B surface antigen by aluminum hydroxide adjuvant", VACCINE, ELSEVIER LTD, GB, vol. 22, no. 11-12, 29 March 2004 (2004-03-29), pages 1475-1479, XP004500392, ISSN: 0264-410X, DOI: 10.1016/J.VACCINE.2003.10.023**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

<u>Field of the Invention</u>

This invention relates to stable vaccine formulations comprising Hepatitis B vaccine phosphoprotein..

<u>Background of the Invention</u>

The loss of a protein's native tertiary structure is generally associated with the loss of its biological activity. It is therefore crucial to ensure that an active protein (e.g. vaccine, therapeutic protein, diagnostic protein etc.) is stored under conditions where the native tertiary structure is maintained.

Storage of proteins for any length of time poses stability problems. The fluctuations of the tertiary structure are proportional to the temperature. Proteins are therefore generally more stable at lower temperatures. Typically, proteins have to be stored freeze-dried (lyophilised) or frozen (around -20°C) to preserve their biological activity. If stored freeze-dried or frozen, the protein has to be reconstituted before its use. For short-term storage of proteins, refrigeration at 4°C may be sufficient.

Proteins are macromolecules consisting of sequences of 20 different naturally occurring amino acids. Seven of these amino acids (aspartic acid, glutamic acid, histidine, cysteine, tyrosine, lysine and arginine) contain a side-chain capable of engaging in acid-base equilibria. This means that they can either accept or donate a proton depending on pH and other species present in the solution. Serine and threonine are also capable of exchanging protons with the surrounding molecules. However, the $pK_a$ values of these amino acids are extremely high (>13.9), so their side-chains are practically always in almost totally protonated form.

The immunogenic activity of protein vaccines depends (to a large extent) on the structural integrity of the key protein antigens, especially in relation to conformational epitopes (where antibodies are required to bind disparate regions of the polypeptide chain brought together by native folding). Irreversible conformational changes and irreversible aggregation lead to inactivation of vaccines. The same considerations apply to proteins adsorbed onto particles, such as alumina particles, or other (non-particulate) surfaces when substantial regions of each protein molecule are still in full interaction with solvent water. This is of particular importance in vaccine distribution in the third world where the maintenance of the cold chain is very difficult or impossible, partly through practical or logistic limitations and partly through cost.

WO2007/003936 (published after the claimed priority dates) discloses an aqueous system comprises a protein and one or more stabilising agents, characterised in that

(i) the one or more stabilising agents have ionisable groups capable of exchanging protons with the protein and with the ionised products of water dissociation;
(ii) the ionisable groups include first groups that are positively charged when protonated and uncharged when deprotonated, and second groups that are uncharged when protonated and negatively charged when deprotonated; and
(iii) the pH of the composition is within a range of protein stability that is at least 50% of the maximum stability of the protein with respect to pH, or within a range of ±0.5 pH units of the pH at which the composition has maximum stability with respect to pH.

WO03/009869 discloses vaccines comprising aluminium adjuvants and histidine.
Jang et al (1997) J Biochem Mol Bio 30(5) 346-351 describes studies on the adsorption of bovine serum albumin or IgG to aluminium adjuvants.

<u>Summary of the Invention</u>

According to the invention, there is provided an aqueous vaccine composition comprising Hepatitis B vaccine phosphoprotein adsorbed on alumina as a solid adsorbent/adjuvant and one or more stabilising agents, wherein the one or more stabilising agents have ionisable groups capable of exchanging protons with the protein and with the ionised products of water dissociation, and wherein the ionisable groups include first groups that are positively charged when protonated and uncharged when deprotonated, and second groups that are uncharged when protonated and negatively charged when deprotonated, wherein the composition comprises phosphate anion at a concentration of greater than 20 mM and wherein the pH of the composition is 5.2 ±0.5 pH units.

The stabilised Hepatitis B vaccine phosphoprotein may be in a microbiologically sterile form, and is conveniently contained or stored in a sealed, sterile container such as a vial, syringe or capsule.

By having a protein at a suitable pH, at or near (within 0.5 pH units, preferably within 0.4 pH units and more preferably within 0.2 pH units) a value at which the proton exchange equilibrium state with respect to its surroundings is optimised for storage stability, so the storage stability of the protein (either at ambient temperature (about 20°C) or at elevated temperature) can be increased, possibly substantially, compared with the storage stability of the protein in non-optimised condition (usually at pH 7).

Storage stability can be further enhanced by use of one or more additives including one or more species capable of exchanging protons with the protein with less extreme $pK_a$ values than those of $H_3O^+$ (which has a $pK_a$ of 13.99) and OH- (which has a $pK_a$ of -1.74). One or more additives with first and second functional groups are used as discussed in more detail, below. This and further discussion may be found in WO2007/003936.

Additives are suitably present in an amount such that the total concentration of additives is in the range 1 mM to 1 M, preferably 1 mM to 200 mM, most preferably 5 mM to 100 mM. In certain practical applications, especially in medical applications, it will often be desirable to use as low concentrations of additives as possible.

Discoveries underlying the present invention have a theoretical basis, as discussed herein, which have been borne out in practice. Indeed, this specification describes how a stabilising system for any protein can be determined, based on available information. Nevertheless, the benefits of the invention may also be achieved on an empirical basis, on the understanding that, for any protein in solution, and depending on the presence of other additives, there is a relationship between stability and pH, and that there is a pH at which the protein has optimum stability. Based on the information presented herein, one of ordinary skill in the art can readily determine additives that are likely to be useful and then whether they in fact meet the criteria by which the invention is defined.

The invention thus enables improvements to be made in the storage stability of Hepatitis B vaccine phosphoprotein in an aqueous environment such that the protein can be stored for extended periods of time at ambient temperatures without significant loss of activity, thus avoiding the need for freezing or refrigeration.

Description of Preferred Embodiments

Certain preferred features of the invention are defined in the subclaims.

It will be appreciated that this invention relates to the stability of Hepatitis B vaccine phosphoprotein particularly the stability of Hepatitis B vaccine phosphoprotein in an aqueous environment, e.g. in aqueous solution, in aqueous gel form and in solid state (or other non-liquid state) where free or bound water is present, and concerns the storage stability of Hepatitis B vaccine phosphoprotein i.e. stability with time, including stability at ambient temperatures (about 20°C) and above.

The application of the invention reduces significantly the probability of irreversible conformational change and irreversible aggregation of Hepatitis B vaccine phosphoprotein and consequent loss of protein activity.

The invention is applicable to stabilisation of Hepatitis B vaccine phosphoprotein throughout its product life including isolation or expression, purification, transport and storage.

The invention is applicable to proteins attached to solid substrates such as vaccine adjuvant by means of hydrophobic, ionic or ligand exchange interactions. The invention is also applicable to proteins in solid state where water has been removed partially or fully from an aqueous solution by drying or by freeze-drying where free or bound water is still present.

The Hepatitis B vaccine phosphoprotein may be native or recombinant, glycosylated or non-glycosylated. The invention is particularly applicable to recombinant Hepatitis B phosphoprotein vaccines. The Hepatitis B vaccine phosphoprotein used in the invention can be maintained substantially in its native state. For the purposes of this specification, the term "native protein" is used to describe a protein having retained tertiary structure, and distinction from proteins that have undergone a degree of unfolding or denaturation. A native protein may incorporate some chemical modification, e.g. deamidation, rather than physical modification.

The Hepatitis B vaccine phosphoprotein is adsorbed on alumina as a solid surface. In some cases, it is beneficial to pre-incubate the solid surface (i.e. alumina particles) in the stabilising formulation prior to adsorption of the Hepatitis B vaccine phosphoprotein onto the surface. This can result in greater stability of the Hepatitis B vaccine phosphoprotein.

For the particular case of Hepatitis B vaccine phosphoprotein , intended for use as a vaccine, and which is therefore used in association with alumina as adsorbent/adjuvant , and particularly in the case where ligand exchange may occur, phosphate is used as stabilising agent. It appears that phosphate may provide additional desirable properties, e.g. when formulations contain phosphate anion (> 20 mM). The reasons behind the importance of phosphate are not entirely clear, but it is believed that phosphate anion plays a role in appropriate binding of the vaccine onto alumina (see, for example, Iyer S. et al.: Vaccine 22 (2004) 1475-1479). As reported in the Example, >95% recovery of antigenic activity was observed of the Hepatitis B vaccine on storage at 55°C for 7 weeks in the formulation based on the novel stabilisation technology in the presence of phosphate anion. Additional experiments were carried out to ensure that the vaccine remained strongly adsorbed on the alumina adjuvant and no changes occurred in the sedimentation rate of the vaccine/alumina particles. Whilst the use of phosphate is very common as a pH buffer in pharmaceutical formulations, its application in the present invention is unusual because the pH of the formulation is 5.2, i.e. a condition where the buffering capacity of phosphate is negligible.

In order to stabilise the protein molecule it is necessary to minimise the frequency and the energy of the proton exchanges at the protein surface. The following three energy-related aspects of the proton exchanges are considered in the present model:

(1) Protein stability can be enhanced by minimising the frequency of the proton exchanges at its surface.
(2) Since the free energy of all protonatable amino acid side-chains is lower in the protonated form than in the de-protonated form, protein stability can be enhanced by keeping as high a proportion of the side-chains as possible in the protonated form.
(3) Since the acid-base dynamic equilibrium of the amino acid side-chains at the surface of a protein is maintained by continuous exchanges of protons with the surrounding molecules, and since the amount of energy released during such proton exchanges depends on the difference in $pK_a$ between the species involved, it is beneficial for the protein stability to substitute the "energetic" proton exchanges (especially those involving $H_3O^+$ and $OH^-$) with "mild" ones (involving chemical species with less extreme $pK_a$ values. This can be achieved by minimising the concentration of $H_3O^+$ and $OH^-$, and Incorporating species capable of exchanging proton with less extreme $pK_a$ values.

*Frequency of proton exchange*

The pKa of a compound is an indication of its acid-base behaviour. The value varies with temperature, and it should be noted that the pKa value of an amino acid side-chain is not necessarily the same as that of the free amino acid (although the difference should not be too great). The pKa values of the side-chains of protein amino acids determine the relative proportion of protonated and de-protonated forms at a given pH. This is shown in Fig 1 of WO2007/003936.
The frequency (or rate) of proton exchange between an amino acid side-chain and surrounding molecules will be maximal if the pH = $pK_a$ of the amino acid. At this pH, the equilibrium is maintained so that there is an equal amount of protonated and de-protonated form of the side-chain. This results in the maximal rate of the protonation/de-protonation cycle. If the pH is remote from the $pK_a$ of the amino acid then the rate of the protonation/de-protonation cycle is reduced by the low proportion of either the protonated form (at high pH) or the de-protonated form (at low pH). For instance, in the case of glutamic acid, the maximal proton exchange frequency will occur at pH around 4.15, in the case of histidine around pH 6 etc. The relative rate of the proton exchanges of the seven amino acids is shown as a function of pH in Fig 2 of WO2007/003936. The relative rate is expressed as the product of the concentrations of the protonated (HA) and the de-protonated (A) form of the amino acid side-chain, with total non-dimensional concentration of the amino acid side-chain arbitrarily selected as 1. The value on the y-axis corresponding to the maximal rate of proton exchange is thus 0.5 times 0.5 = 0.25.
The protein stability can be enhanced by minimising the overall frequency of the proton exchanges, i.e. the sum of all proton exchange events at the protein surface. An example of the overall frequency of proton exchange frequency is shown in Fig 3 of WO2007/003936, for the protease enzyme papain. This was calculated by summing the proton exchange frequencies of the individual amino acids while the relative contribution of each amino acid was determined by its the relative abundance in the papain sequence. The pH optimum for papain stability was found experimentally to be between 5.8 and 6.2. This corresponds approximately to the minimum of the proton exchange rate.
However, the relative frequency of the proton exchange is only one of the contributing factors to the protein stability/instability and has to be considered in the context of other contributing factors (see below). This means that, whilst the frequency of the proton exchange is a good indication of the pH optimum, the highest stability is not necessarily attained precisely in the minimum of the proton exchange frequency profile.

*Minimising free (Gibbs) energy of the side-chains*

The Gibbs free energy of all protonatable amino acid side-chains is lower in the protonated form than in the de-protonated form. This is because the free electron pairs (onto which the proton binds) are lower in energy in the presence of the proton compared with its absence. Binding of protons onto the amino acid side-chains thus decreases overall free energy of the entire protein. The protein stability can therefore be enhanced by maximising the protonation of the amino acids on its surface.
The percentage of overall protonation at the surface of two model proteins (glucose oxidase and papain) as a function of pH is shown in Fig 4 of WO2007/003936. This was calculated by summing the proportion of the protonated form of all protonatable amino acids in the sequence of the two proteins. Whilst glucose oxidase is an example of a protein with low isoelectric point (pl = approx. 4.4), papain is an enzyme with very high isolelectric point (pl = approx. 8.7). This is reflected in the protonation profile. The percentage of side-chain protonation at neutral pH (~7) is considerably higher in the case of papain than in the case of glucose oxidase. There will therefore be a greater tendency to push the pH optimum towards lower pH in the case of glucose oxidase than in the case of papain.

It was found experimentally that the actual pH optimum for the enzyme stability is always at least slightly (or more considerably) away towards low pH from the theoretical pH optimum calculated on the basis of the proton exchange frequency. The degree of protonation is apparently the crucial parameter that determines how far towards the low pH the optimum is shifted from the theoretical pH optimum calculated on the basis of the proton exchange frequency. Since the protonation degree is closely linked to the pl of the protein this effect can be summarised as follows: whilst proteins with high pl will only have a small tendency to shift their actual pH optimum towards low pH the proteins with low pl will have a greater tendency to shift the pH optimum in this direction.

*Minimising the energy released during the proton exchanges*

The amount of energy released during the proton exchanges depends on the difference in $pK_a$ between the species involved. It is therefore beneficial for the stability of the protein to minimise the impact of continuous interactions with compounds of extreme $pK_a$ (especially those involving $H_3O^+$ and $OH^-$) and ensure that these are replaced by interactions with compounds with less extreme $pK_a$ values.

The dominant species with "extreme $pK_a$ values" in aqueous solutions are $H_3O^+$ and $OH^-$. The more extreme is the $pK_a$ value of a species, the less likely it is that this species occurs in the "high energy" form (i.e. protonated form in the case of a strong acid or the deprotonated form in the case of strong base). It can be said that, the more harmful the species is for the protein, the less likely it is to occur in the aqueous solution, this being a useful feedback mechanism protecting the protein. Such feedback mechanism also applies to $H_3O^+$ and $OH^-$. However, these two species are derived from water (by protonation or de-protonation) whose concentration in aqueous solutions is extremely high (-55.5 M) which, in turn, increases the relative abundance of these "high energy" species. Therefore, given the extreme $pK_a$ of $H_3O^+$ and OH- the concentration of these species will always be very high (e.g. three orders of magnitude higher than concentration of other species of comparable $pK_a$ derived from an additive present at 55.5 mM concentration).

The concentrations of $H_3O^+$ and OH- in aqueous solutions at 25°C of given pH are shown in Fig 6 of WO2007/003936. There would be some logic in asserting that the optimum pH for protein stability must be 7, because this is the pH where the total concentration of $H_3O^+$ and OH- is at its minimum (200 nM). For comparison, the total concentration of $H_3O^+$ and OH- at pH 6 or 8 is 1.1 $\mu$M, at pH 5 or 9 it is 10.01 $\mu$M and so on. Nevertheless, this would only be the case if the concentration of $H_3O^+$ and OH-was the only parameter determining the probability of destructive proton exchanges. The probability also depends on the protonation status of the protein at the given pH, as discussed above. Furthermore, it depends on the overall amino acid composition of the protein, which determines whether the protein is overall more susceptible to the $H_3O^+$ attack or the $OH^-$ attack. The pH optimum can thus be quite remote from 7.

The energy impact of the proton exchanges between the amino acid side-chains and $H_3O^+$ or OH- is indicated in Table 1. The impact is expressed as the difference in $pK_a$ between the colliding species (the difference in $pK_a$ being proportional to the energy released during the collision).

**Table 1**

| Amino acid | $pK_a$ | $pK_a$ - $pK_{H3O+}$ | $pK_{OH-}$ - $pK_a$ |
|---|---|---|---|
| Aspartic acid | 3.71 | 5.45 | 10.28 |
| Glutamic acid | 4.15 | 5.89 | 9.84 |
| Histidine | 6.04 | 7.78 | 7.95 |
| Cysteine | 8.14 | 9.88 | 5.85 |
| Tyrosine | 10.10 | 11.84 | 3.89 |
| Lysine | 10.67 | 12.41 | 3.32 |
| Arginine | 12.1 | 13.84 | 1.89 |

Whilst in the case of the more acidic amino acids (aspartic acid, glutamic acid and histidine) it is the interaction with $OH^-$ that results in more energy release, in the case of the more alkaline amino acids it is the interaction with the $H_3O^+$ that is more destabilising.

Because of the tendency of the protein to keep as many amino acid side-chains as possible in the low energy protonated form, it can be assumed that the three most alkaline side-chains (tyrosine, lysine and arginine - all with $pK_a$ > 10) will be virtually fully protonated in the optimum conditions and their engagement in the proton exchange will therefore be minimal. This assumption is in accord with experimental results: the pH optima of the model proteins tested ranged between 4.8 - 8.0. Even at pH 8.0 tyrosine, lysine and arginine are almost fully protonated. It is therefore the relative abundance of cysteine, histidine, glutamic acid and aspartic acid that determines the susceptibility of the protein to $H_3O^+$

or OH⁻ and thus the optimum conditions for the protein stability.

There will be a tendency of the proteins with low pI to push their pH optimum towards lower values. This is because the low pI proteins contain a higher proportion of the most acidic amino acids (aspartic acid and glutamic acid), which are particularly susceptible to OH⁻ attack (see Table 1).

The pH adjustment is not the only measure that can be taken to improve the protein stability. The stability can be further improved by adding compounds that can engage in "mild" proton exchanges with the amino acid side-chains of the protein and thus reduce further the probability of the destructive "energetic" proton exchanges with $H_3O^+$ or OH⁻.

It was found experimentally that the best results can be achieved if two acid-base functional groups are incorporated. Preferably, the first functional group has a $pK_a$ at least one unit higher than the pH optimum and is positively charged at the pH optimum. This means that the charge of the group switches from neutral to positive while accepting the proton (e.g. amino group, purine, TRIS etc.). The second functional group preferably has a $pK_a$ at least one unit lower than the pH optimum and is negatively charged at the pH optimum. This means that the charge of the group switches from negative to neutral while accepting the proton (e.g. carboxylic group).

The incorporation of the additives can affect slightly the pH optimum for the protein stability (within approximately 0.5 pH unit depending on the nature and quantity of the additives).

It was also found that a relative excess of the first group (i.e. the positively charged at the pH optimum) is beneficial for the protein stability. This can be speculatively explained by forming ionic bonds with the negatively charged side-chains of aspartic acid and glutamic acid; the pH optimum would have to be far below 4 in order for these side-chains to become largely protonated. Such ionic bonds might lower the Gibbs energy of the free electron pair of these side-chains, just as binding of a proton does.

An algorithm has been derived, to allow a good prediction of the pH optimum based on the known amino acid sequence of the protein and its isoelectric point. Whilst the prediction can be achieved using the total amino acid sequence, it is recommended to only take into account the amino acid side-chains that are accessible at the surface of the protein. Both the total amino acid sequences and the accessibility of the individual amino acids in the protein structure can be found using the Protein Data Bank information database (http://www.rcsb.org/pdb). For the amino acid accessibility estimation it is necessary to download the DEEP VIEW protein software (available at http://www.expasy.org/spdbv)

The algorithm consists of three steps as follows

**Step 1**: (this can be done easily in MS-Excel)

Calculate the proton exchange frequency function (P) and plot against pH:

$$P = \frac{100000}{M} \times \sum_{1}^{7}(N \times [HA] \times [A])$$

where

$$[HA] = \frac{1}{1 + \frac{10^{-pKa}}{10^{-pH}}} \qquad [A] = \frac{10^{-pKa}}{10^{-pKa} + 10^{-pH}}$$

M is the relative molecular weight of the protein unit
N is the number of the given amino acid side-chains in the protein unit (1 = aspartic acid, 2 + glutamic acid, 3 = histidine etc.)
[HA] is the proportion of the protonated form of the amino acid at the given pH. 0<[HA]<1.
[A] is the proportion of the de-protonated form of the amino acid at the given pH. 0<[A]<1.

If plotted against pH, the proton exchange frequency profile can be obtained;

**Step 2**: Calculate the magnitude of the pH shift (denoted here as X) as follows:

$$X = A \times pI + B$$

where A = -1.192 and B = 10.587 if the total amino acid composition is used; and

A = -0.931 and B = 8.430 if only the amino acids accessible at the protein surface is used.

Find minimum of the function obtained in step 1 in the pH interval 4-9. Add the calculated pH shift value to the minimum to obtain value Y:

$$Y = P_{minimum} + X$$

Read the pH that corresponds to the value Y in the P vs. pH graph. There will always be two pH values that correspond to the value Y in the P vs. pH graph (one below the Minimum and one above the $P_{minimum}$. It is important that the value is read below the $P_{minimum}$ (i.e. toward the lower pH values). This is the estimated pH optimum for the stability of the enzyme. The relation between parameters X, Y and $P_{minimum}$ is demonstrated in Fig 7 of WO2007/003936.

**Step 3**: Once the pH optimum is selected, one or more additives that contain one or more of the following functional groups may be chosen.

Functional group 1:

The first functional group has a $pK_a$ higher than the pH optimum for the protein stability (as estimated in steps 1 and 2) and is positively charged at the pH optimum. This means that the charge of the group switches from neutral to positive while accepting the proton.

The $pK_a$ of the functional group must be higher than the pH optimum for the protein stability. Preferably, it should be within 5 pH units above the pH optimum. More preferably, it should be within 0.5 - 4 pH units above the pH optimum. Most preferably, it should be within 1 - 3 pH units above the pH optimum for the protein.

Functional group 2:

The second functional group has a $pK_a$ at least one unit lower than the pH optimum for the protein stability (as estimated in steps 1 and 2) and is negatively charged at the pH optimum. This means that the charge of the group switches from negative to neutral while accepting the proton (e.g. carboxylic group).

The $pK_a$ of the functional group must be lower than the pH optimum for the protein stability. Preferably, it should be within 5 pH units below the pH optimum. More preferably, it should be within 0.5 - 4 pH units above the pH optimum. Most preferably, it should be within 1 - 3 pH units below the pH optimum for the protein.

The formulation can only contain the first functional group (i.e. the positively charged one). Preferably, the formulation contains both the first and the second functional group.

The two functional groups can be contained within one compound (e.g. an amino acid). Preferably, the two functional groups are located on more than one additive (e.g. one additive contains the first, negatively charged group, and the other additive contains the second, positively charged group). There is no limit to the number of each of the two types of functional groups added to the formulation.

In some cases it may be beneficial to incorporate the positively charged functional group in excess relative to the amount of the negatively charged group.

The buffering ability of the additives can be sufficient in those cases where their $pK_a$ is only about 1 pH unit from the pH optimum. If the $pK_a$ is further away their buffering ability may be reduced. A small concentration of buffer with $pK_a$ close to the pH optimum can then be used in order to maintain the required pH.

Some examples of classes of groups for each of the two functional groups that can be usefully incorporated as the stabilising additives is shown in Table 2.

**Table 2**

| Functional group 1 | Functional group 2 |
|---|---|
| $\alpha$-Amino group of amino acids | $\alpha$-Carboxylic group of amino acids |
| Side-chain amino group of amino acids | Side-chain carboxylic group of amino acids |
| Purine (and purine based compounds) | Carboxylic group of organic acids such as: |
| Primary amines | Lactic acid |
| Secondary amines | Succinic acid |

7

(continued)

| Functional group 1 | Functional group 2 |
|---|---|
| Tertiary amines | |
| Quaternary amines such as TRIS | Inorganic acids |

A list of specific examples of possible additive components, with pK$_a$ values, is given in Table 3.

Table 3

| Amino Acids | pK$_a$ of functional group 1 | pK$_a$ of functional group 2 |
|---|---|---|
| Glycine | 9.8 | 2.4 |
| Alanine | 9.9 | 2.4 |
| Valine | 9.7 | 2.2 |
| Leucine | 9.7 | 2.3 |
| Isoleucine | 9.8 | 2.3 |
| Serine | 9.2 | 2.2 |
| Threonine | 9.1 | 2.1 |
| Cysteine | 10.8, 8.3 | 1.9 |
| Methionine | 9.3 | 2.1 |
| Aspartic acid | 9.9 | 2.0, 3.7 |
| Asparagine | 8.8 | 2.1 |
| Glutamic acid | 9.5 | 2.1, 4.1 |
| Glutamine | 9.1 | 2.2 |
| Arginine | 9.0 | 1.8 |
| Lysine | 9.2 | 2.2 |
| Histidine | 9.2, 6.0 | 1.8 |
| Phenylalanine | 9.2 | 2.2 |
| Tyrosine | 9.1 | 2.2 |
| Tryptophan | 9.4 | 2.4 |
| Proline | 10.6 | 2.0 |
| Ornithine | 8.69 | 1.7 |
| Citrulline | 9.69 | 2.4 |
| & various peptides consisting of the above amino acids | | |
| **Other carboxylic acids** | | |
| Formic acid | | 3.8 |
| Glyoxylic acid | | 3.2 |
| Oxalic acid | | 1.3, 4.2 |
| Acetic acid | | 4.8 |
| Glycolic acid | | 3.8 |
| Pyruvic acid | | 2.4 |

(continued)

| Other carboxylic acids | | |
|---|---|---|
| Malonic acid | | 2.8, 5.7 |
| Lactic acid | | 3.8 |
| Glyceric acid | | 3.5 |
| Fumaric acid | | 3.0, 4.4 |
| Succinic acid | | 4.2, 5.6 |
| Malic acid | | 3.4, 5.1 |
| $\alpha$-Tartaric acid | | 3.0, 4.3 |
| Glutaric acid | | 4.3, 5.4 |
| Ascorbic acid | | 4.1 |
| Adipic acid | | 4.4 |
| Gallic acid | | 4.4 |
| **Amines** | | |
| Trimethylamine | 9.8 | |
| 1,2-Propanediamine | 6.66, 9.8 | |
| 1,3-Propanediamine | 9.0, 10.3 | |
| 1,2,3-Triaminopropane | 9.5, 7.9 | |
| Pentylamine | 10.6 | |
| Tris(hydroxymethyl)aminomethane | 8.1 | |
| Benzylamine | 9.3 | |
| Phenyl ethylamine | 9.8 | |
| Tyramine | 9.7 | |
| Tryptamine | 10.2 | |
| Hydroxytryptamine | 9.8 | |
| **Imines** | | |
| Ethyleneimine | 8.01 | |
| **Other compounds** | | |
| Purine | 8.96 | |
| 8-hydroxypurine | 8.26 | |
| Ammonium ion | 9.25 | |
| Quinoline | 4.9 | |
| 1-Isoquinolineamine | 7.6 | |
| 1-Methylimidazol | 7.0 | |
| Allantoin | 8.9 | |
| Veronal | 7.4 | |
| 2-Ethylbenzimidazole | 6.2 | |

(continued)

| Other compounds | | |
|---|---|---|
| Brucine | 8.3 | |
| Uracil | 9.5 | |
| 2,4,6-Trimethylpyridine | 7.5 | |

The materials listed above are given for the purpose of illustration only. It will of course be understood by one of ordinary skill in the art that aspects specific to the particular protein have to be taken into account. For instance, it is important to ensure that the additives selected do not inhibit the protein activity. It is also important to ensure that the compounds used to improve heat stability of proteins are themselves stable under the conditions employed.

The invention can be combined with other well established approaches to protein stability. For example, a protease inhibitor can be incorporated in the formulation to ensure that the protein is not slowly digested by protease activity present in the sample.

Another additive that may be used is a polyalcohol, e.g. at a concentration of at least 0.5%, and typically up to 5% (w/w). Examples of such compounds are saccharides such as inositol, lactitol, mannitol, xylitol and trehalose.

The ionic strength of the protein formulation stabilised by application of the present invention can be adjusted to meet the requirement for the intended use of the formulation (e.g. isotonic formulation for therapeutic use). Importantly, experiments showed repeatedly that in principle the stability of proteins at room temperature mirrors that at higher temperature, the rate of activity decline being many orders of magnitude slower at room temperature compared with that at increased temperature (e.g. 60°C).

The following Example illustrates the invention.

Example - Hepatitis B recombinant vaccine

The *in vitro* antigenic activity of the Hepatitis B vaccine was measured using the AUSZYME monoclonal diagnostic kit (Abbott Laboratories; cat no. 1980-64). The antigenic activity was determined both in the whole vaccine and in the supernatant following centrifugation (13,000 RPM, 5 min). Each sample was measured in triplicate. The antigenic activity was expressed as a percentage with respect to the value measured of the untreated refrigerated vaccine as follows:

$$R = \frac{(S - N)}{(C - N)} \times 100$$

$$S = \frac{S1 + S2 + S3}{3}$$

$$C = \frac{C1 + C2 + C3}{3}$$

where:

R is the recovery of antigenic activity (%)
N is the value of the negative control AUSZYME measurement
C1, C2 and C3 are the values of three repeated AUSZYME measurements of the Control sample (i.e. untreated refrigerated vaccine)
S1, S2 and S3 are the values of three repeated AUSZYME measurements of the tested sample.

The remaining antigenic activity of Hepatitis B vaccine was tested after incubation at 55°C for 2, 4 and 7 weeks. The stabilising formulations consisting of histidine and phosphate anion and adjusted to the optimum pH retained >95% of the original antigenic activity after 7 weeks. In contrast, the remaining antigenic activity of the original vaccine formulation (18 mM phosphate buffer, pH 6.9-7.1, containing 132 mM sodium chloride) was < 10% at this point. The stabilising formulations did not appear to affect the alumina-antigen association as the antigenic activity measured in the supernatant of the stabilising formulation following centrifugation was comparable with that measured in the supernatant of the original sample. It is believed that the presence of phosphate anion ensures optimal binding of the vaccine onto the alumina.

### Claims

1. An aqueous vaccine composition comprising Hepatitis B vaccine phosphoprotein adsorbed on alumina as a solid adsorbent/adjuvant and one or more stabilising agents, wherein the one or more stabilising agents have ionisable groups capable of exchanging protons with the protein and with the ionised products of water dissociation, and wherein the ionisable groups include first groups that are positively charged when protonated and uncharged when deprotonated, and second groups that are uncharged when protonated and negatively charged when deprotonated, wherein the composition comprises phosphate anion at a concentration of greater than 20 mM and wherein the pH of the composition is 5.2 ±0.5 pH units.

2. A composition according to claim 1, wherein the first and second groups have pKa values respectively higher and lower than the pH of the composition and are at least 50% of these groups are ionised.

3. A composition according to claim 2, wherein at least 80% of the groups are ionised, and preferably the groups are substantially completely ionised.

4. A composition according to claim 2 or claim 3, wherein the respective pKa values are each within 0.5 to 4 pH units of the pH of the composition.

5. A composition according to claim 4, wherein the respective pKa values are each within 1 to 3 pH units of the pH of the composition.

6. A composition according to any preceding claim, which additionally comprises a polyalcohol.

7. A composition according to any preceding claim, which comprises at least 0.1% (w/w) of the one or more stabilising agents.

8. A composition according to any preceding claim, which comprises up to 200 mM of the or each stabilising agent.

9. A composition according to any preceding claim, which comprises up to 100 mM, of the or each stabilising agent.

10. A composition according to any preceding claim, wherein the Hepatitis B vaccine phosphoprotein is in the native state.

11. A composition according to any preceding claim, for use in therapy practised on the human or animal body.

12. A substantially dry formulation obtainable by drying a composition according to any of claims 1 to 11, and which can be reconstituted.

### Patentansprüche

1. Wässrige Impfstoffzusammensetzung, umfassend Hepatitis-B-Impfstoff-Phosphoprotein, adsorbiert an Aluminiumoxid als festes Adsorbens/Adjuvans, sowie ein oder mehrere Stabilisierungsmittel, wobei das eine bzw. die mehreren Stabilisierungsmittel ionisierbare Gruppen aufweist bzw. aufweisen, die mit dem Protein und mit den ionisierten Produkten der Wasserdissoziation Protonen austauschen können, und wobei die ionisierbaren Gruppen erste Gruppen, die im protonierten Zustand positiv geladen und im deprotonierten Zustand ungeladen sind, und zweite Gruppen, die im protonierten Zustand ungeladen und im deprotonierten Zustand negativ geladen sind, umfassen, wobei die Zusammensetzung Phosphatanion in einer Konzentration von mehr als 20 mM umfasst und wobei der pH-Wert der Zusammensetzung 5,2 ±0,5 pH-Einheiten beträgt.

2. Zusammensetzung nach Anspruch 1, wobei die ersten und zweiten Gruppen pKa-Werte aufweisen, die oberhalb bzw. unterhalb des pH-Werts der Zusammensetzung liegen, und wobei wenigstens 50% dieser Gruppen ionisiert sind.

3. Zusammensetzung nach Anspruch 2, wobei wenigstens 80% der Gruppen ionisiert sind und wobei vorzugsweise die Gruppen im Wesentlichen vollständig ionisiert sind.

4. Zusammensetzung nach Anspruch 2 oder Anspruch 3, wobei die jeweiligen pKa-Werte jeweils um nicht mehr als 0,5 bis 4 pH-Einheiten vom pH-Wert der Zusammensetzung abweichen.

5. Zusammensetzung nach Anspruch 4, wobei die jeweiligen pKa-Werte jeweils um nicht mehr als 1 bis 3 pH-Einheiten vom pH-Wert der Zusammensetzung abweichen.

6. Zusammensetzung nach einem vorhergehenden Anspruch, die zusätzlich einen Polyalkohol umfasst.

7. Zusammensetzung nach einem vorhergehenden Anspruch, die wenigstens 0,1 Gew.-% der ein oder mehreren Stabilisierungsmittel umfasst.

8. Zusammensetzung nach einem vorhergehenden Anspruch, die bis zu 200 mM des bzw. eines jeden Stabilisierungsmittels umfasst.

9. Zusammensetzung nach einem vorhergehenden Anspruch, die bis zu 100 mM des bzw. eines jeden Stabilisierungsmittels umfasst.

10. Zusammensetzung nach einem vorhergehenden Anspruch, wobei das Hepatitis-B-Impfstoff-Phosphoprotein im nativen Zustand vorliegt.

11. Zusammensetzung nach einem vorhergehenden Anspruch, zur Verwendung bei der therapeutischen Behandlung des menschlichen oder tierischen Körpers.

12. Im Wesentlichen trockene Formulierung, erhältlich durch Trocknen einer Zusammensetzung nach einem der Ansprüche 1 bis 11, wobei sich die Formulierung rekonstituieren lässt.


**Revendications**

1. Composition de vaccin aqueuse comprenant la phosphopratéine, adsorbée sur alumine, du vaccin de l'hépatite B en tant qu'adsorbant solide/adjuvant et un ou plusieurs agents stabilisants, l'un ou plusieurs agents stabilisants portant des groupements ionisables susceptibles d'échanger des protons avec la protéine et avec les produits ionisés de la dissociation de l'eau, et les groupes ionisables incluant des premiers groupements qui sont chargés positivement lorsqu'ils sont protonés et non chargés lorsqu'ils sont déprotonés, et des seconds groupements qui sont non chargés lorsqu'ils sont protonés et chargés négativement lorsqu'ils sont déprotonés, la composition comprenant l'anion phosphate à une concentration supérieure à 20 mM et le pH de la composition étant de 5,2±0,5 unités de pH.

2. Composition selon la revendication 1, les premiers et seconds groupements possédant des valeurs de pKa respectivement supérieures et inférieures au pH de la composition et au moins 50 % de ces groupements étant ionisés.

3. Composition selon la revendication 2, au moins 80 % des groupements étant ionisés, et préférablement les groupements étant substantiellement complètement ionisés.

4. Composition selon la revendication 2 ou la revendication 3, dans laquelle les valeurs respectives de pKa sont chacune comprises dans la fourchette de 0,5 à 4 unités de pH du pH de la composition.

5. Composition selon la revendication 4, dans laquelle les valeurs respectives de pKa sont chacune comprises dans la fourchette de 1 à 3 unités de pH du pH de la composition.

6. Composition selon l'une quelconque des revendications précédentes, qui de plus comprend un polyol.

7. Composition selon l'une quelconque des revendications précédentes, qui comprend au moins 0,1 % en poids de l'un ou des plusieurs agents stabilisants.

8. Composition selon l'une quelconque des revendications précédentes, qui comprend jusqu'à 200 mM du ou de chaque agent stabilisant.

9. Composition selon l'une quelconque des revendications précédentes, qui comprend jusqu'à 100 mM du ou de chaque agent stabilisant.

10. Composition selon l'une quelconque des revendications précédentes, la phosphoprotéine du vaccin de l'hépatite B étant dans l'état natif.

11. Composition selon l'une quelconque des revendications précédentes, destinée à être utilisée en thérapie pratiquée sur le corps humain ou animal.

12. Formulation substantiellement sèche que l'on peut obtenir par séchage d'une composition selon l'une quelconque des revendications 1 à 11, et qui peut être reconstituée.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007003936 A

- WO 03009869 A

**Non-patent literature cited in the description**

- **JANG et al.** *J Biochem Mol Bio,* 1997, vol. 30 (5), 346-351

- **LYER S. et al.** *Vaccine,* 2004, vol. 22, 1475-1479